(19) Europäisches Patentamt
European Patent Office
Office européen des brevets

(11) **EP 3 006 012 A1**

(12) **EUROPEAN PATENT APPLICATION**
published in accordance with Art. 153(4) EPC

(43) Date of publication:
**13.04.2016 Bulletin 2016/15**

(51) Int Cl.:
**A61K 8/19** (2006.01)    **A61K 8/25** (2006.01)
**A61Q 11/00** (2006.01)

(21) Application number: **14808142.5**

(22) Date of filing: **05.06.2014**

(86) International application number:
**PCT/JP2014/064933**

(87) International publication number:
**WO 2014/196591 (11.12.2014 Gazette 2014/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **07.06.2013   JP 2013120778**

(71) Applicant: **Sunstar Suisse, S.A.**
**1163 Etoy (CH)**

(72) Inventors:
• **HASEGAWA, Noritaka**
  **Takatsuki-shi**
  **Osaka 569-1044 (JP)**
• **TACHIBANA, Aki**
  **Takatsuki-shi**
  **Osaka 569-1044 (JP)**

(74) Representative: **TBK**
**Bavariaring 4-6**
**80336 München (DE)**

(54) **COMPOSITION FOR ORAL USE CONTAINING DIAMOND PARTICLES**

(57)     An object of the present invention is to provide a composition for oral use having excellent cleaning ability and smoothing. The present invention provides a composition for oral use containing diamond particles and silica particles.

**Description**

Technical Field

The present invention relates to a composition for oral use containing diamond particles, and more specifically to a composition for the oral cavity, comprising diamond particles and silica particles. This application claims priority to Japanese Patent Application No. 2013-120778 filed on June 7, 2013. The disclosure in the specification and/or the drawings of the patent application is incorporated herein by reference.

Background Art

Tooth discoloration results from deposition on the teeth of chromogenic substances called "stains." This causes a serious problem in terms of aesthetics. As a means for removing stains, a common method is that a composition for oral use, such as a dentifrice composition containing a dental abrasive, is held in the mouth with a tool such as a toothbrush, and the teeth are brushed. The composition for oral use is expected to not only efficiently remove stains but also make the tooth surfaces smooth after use, thereby making it difficult for stains or the like to adhere to the teeth and thus providing an effect of keeping the teeth beautiful.
It is generally thought that a composition for oral use comprising a dental abrasive with a higher abrasive power has greater cleaning ability and smoothing. However, when the abrasive power is high, there is a risk that the abrasion on the teeth may be more than necessary. In this case, although stains may be removed, the teeth themselves will wear away over time. When the tooth surface wears away too much, the dentin may be exposed, which may cause hypersensitivity. Further, because the exposed dentin has poor resistance to acid, tooth decay may be promoted. Further, even if a composition has high abrasive power, it is difficult to predict whether the composition can make the tooth surface smooth, and it may cause more abrasion than necessary on the tooth surface, resulting in a rough surface.
As described above, the composition for oral use must have stain removal ability (hereinafter also referred to as "cleaning ability") and the ability to make the tooth surface smooth (hereinafter also referred to as "smoothing").

Citation List

Patent Literature

PTL 1: WO 10/060653

Summary of Invention

Technical Problem

An object of the present invention is to provide a composition for oral use that has excellent cleaning ability and smoothing.

Solution to Problem

The present inventors found that a composition for oral use comprising diamond particles and silica particles has excellent cleaning ability and smoothing. The inventors completed the present invention with further improvements based on this finding.
Specifically, the present invention encompasses, for example, the subjects described in the following items.
Item 1. A composition for oral use comprising diamond particles and silica particles.
Item 2. The composition for oral use according to Item 1, wherein the diamond particles have an average particle size (d50) of 0.5 to 5 $\mu$m.
Item 3. The composition for oral use according to item 1, wherein the silica particles have an average particle size (d50) of 0.5 to 20 $\mu$m.
Item 4. The composition for oral use according to any one of Items 1 to 3, wherein the diamond particles are present in an amount of 0.01 to 0.5 mass%.
Item 5. The composition for oral use according to any one of Items 1 to 4, wherein the silica particles comprise a combination of first silica particles and second silica particles, the first silica particles having an average particle size (d50) of 3 to 5.5 $\mu$m and an oil absorption of 20 to 80 (mL/100 g), and second silica particles having an average particle size (d50) of 6 to 15 $\mu$m and an oil absorption of 90 to 150 (mL/100 g).
Item 6. The composition for oral use according to any one of Items 1 to 5, wherein the silica particles are present in an amount of 1 to 35 parts by mass per 0.01 to 0.5 parts by mass of the diamond particles.

Advantageous Effects of Invention

Treating (for example, brushing) the teeth with the composition for oral use of the present invention can efficiently remove stains and also make the tooth surfaces smooth after use to keep the teeth beautiful. Specifically, the composition for oral use of the present invention has excellent cleaning ability and smoothing.

Description of Embodiments

The present invention is described in more detail below.

The composition for oral use of the present invention comprises diamond particles and silica particles.

The diamond particles preferably have an average particle size (particle diameter corresponding to 50% in the cumulative particle size distribution (i.e., 50% cumulative diameter): d50) of 0.5 to 5 $\mu$m, more preferably 1 to 4 $\mu$m, and even more preferably 2 to 3 $\mu$m.

Further, the diamond particles preferably have a 99% cumulative diameter (d99) of 7 $\mu$m or less, more preferably 5 $\mu$m or less, and even more preferably 4.5 $\mu$m or less.

Further, the diamond particles preferably have a 10% cumulative diameter (d10) of 0.1 $\mu$m or more, more preferably 0.5 $\mu$m or more, even more preferably 1 $\mu$m or more, and still more preferably 1.8 $\mu$m or more.

With respect to the above conditions (preferable ranges) of d50, d99, and d10, diamond particles that satisfy the conditions of d50 are preferable. Diamond particles that satisfy the conditions of d50 and d99 are more preferable, and diamond particles that satisfy the conditions of d50, d99, and d10 are even more preferable.

As such diamond particles, natural or synthetic products can be used. Synthetic products can be produced, for example, by a known method, such as a high-temperature, highpressure synthesis method, a chemical vapor deposition method, a detonation synthesis method, or an ultrasonic cavitation method.

The cumulative diameter of the diamond particles as referred to herein is a value determined based on the Coulter Principle (the electrical sensing zone method) using water as a dispersion (i.e., a value obtained using a Coulter counter). This value can be measured, for example, by using a Multisizer 3 Coulter (produced by Beckman Coulter, Inc.)

The silica particles contained in the composition for oral use of the present invention may be, for example, silica known to be used for compositions for oral cavity and synthesized by a wet method or a dry method. Since the balance between cleaning ability and abrasiveness of silica synthesized by wet methods is suitable for use in the present invention, silica synthesized by wet methods is preferable. Examples of wet methods include precipitation methods and gel methods. Silica obtained by wet methods can be roughly classified into precipitated silica synthesized by precipitation methods and gelled silica synthesized by gel methods. In the present invention, any type of silica can be used. Precipitated silica is often more preferable than gelled silica, but this is not limitative. The phrase "often more preferable" means that precipitated silica often has preferable values in terms of the average particle size and oil absorption described below, and BET specific surface area.

Commercially available silica products can be used as silica particles used in the present invention. Examples of precipitated silica particles include Aerosil, Ultrasil, Carplex (all produced by Evonik Degussa), Aerosil (produced by Japan Aerosil Co., Ltd.), Zeodent (produced by Huber Corporation), Excelia, Reolosil (both produced by Tokuyama Corporation), Syilsia (produced by Fuji Sylsia Chemical Ltd.), Sorbosil (produced by Ineos Silicas Ltd.), Denka fused silica (produced by Denki Kagaku Kogo Kabushiki Kaisha), Nipsil and Nipgel (both produced by Tosoh Silica Corporation), Zeosil, Tixosil (produced by Solvay S.A.), and the like. Among these, for example, Zeodent (produced by Huber Corporation), Sorbosil AC (produced by PC Corporation), and Texosil (produced by Phodia Co., Ltd.), all of which are precipitated silica particles, are preferable.

The silica particles preferably have an average particle size (d50) of 0.5 to 20 $\mu$m, more preferably 1 to 15 $\mu$m, even more preferably 2 to 13 $\mu$m, and still even more preferably 3.5 to 13 $\mu$m. The silica particles preferably have an oil absorption (linseed-oil absorption according to JIS K5101) of 10 to 200 (mL/100 g), more preferably 20 to 150 (mL/100 g), even more preferably 25 to 140 (mL/100 g), and still even more preferably 30 to 135 (mL/100 g).

The silica particles contained in the composition for oral use of the present invention preferably comprise a combination of silica particles having different properties. Although a combination of two or more types (for example, three, four, or five types) of silica particles may be used, using a combination of two types of silica particles having different properties is the most preferable. Examples of the combination of two types of silica particles having different properties are shown below. When the two types of silica particles used in combination are termed "first silica particles" and "second silica particles," respectively, the first silica particles preferably have an average particle size (d50) of 3 to 5.5 $\mu$m (more preferably in the range of 3.5 to 5 $\mu$m), and an oil absorption (linseed oil absorption according to JIS K5101) of 20 to 80 (mL/100 g) (more preferably in the range of 30 to 70 (mL/100 g)). The second silica particles preferably have an average particle size (d50) of 6 to 15 $\mu$m (more preferably in the range of 6 to 12 $\mu$m) and an oil absorption (linseed oil absorption according to JIS K5101) of 90 to 150 (mL/100 g) (more preferably in the range of 90 to 140 (mL/100 g)).

The average particle size (d50) of silica referred to herein is a value determined by laser diffraction scattering using water as a dispersant. The value can be determined, for example, by using a Malvern Mastersizer based on the principle of Fraunhofer diffraction using a low-power He/Ne laser.

The composition for oral use of the present invention preferably comprises diamond particles in an amount of 0.01 to 0.5 mass%, and more preferably 0.05 to 0.2 mass%. The composition for oral use of the present invention preferably comprises the silica particles in an amount of 1 to 35 mass%, and more preferably 2 to 30 mass%.

The composition for oral use of the present invention preferably comprises the silica particles in an amount of 1 to 35 parts by mass, and more preferably 5 to 30 parts by mass, per 0.01 to 0.5 parts by mass (preferably 0.05 to 0.2 parts by mass) of the diamond particles. In particular, the silica particles are more preferably used in an amount of 15 to 30 parts by mass per 0.01 to 0.5 parts by mass of the diamond particles. The silica particles are particularly preferably used in an amount of 20 to 30 parts by mass per 0.05 to 0.2 parts by mass of the diamond particles.

When a combination of the first silica particles and second silica particles is used as the silica particles, the second silica particles are preferably used in an amount of 0.5 to 25 parts by mass, more preferably 1 to 20 parts by mass, even more preferably 1 to 10 parts by mass, still more preferably 1 to 5 parts by mass, even still more preferably 1.5 to 4.5 parts by mass, particularly preferably 2 to 4 parts by mass, and most preferably 2.5 to 3.5 parts by mass, per part by mass of the first silica particles.

The amount of the first silica particles in the composition is preferably 2 to 8 mass%, and more preferably 3 to 7 mass%, and the amount of the second silica particles in the composition is preferably 2 to 25 mass%, and more preferably 5 to 22 mass%.

The composition for oral use of the present invention can be produced by using a known method. The composition for oral use of the present invention can be used for teeth and dentures, and can be formed into dentifrices in various forms by usual methods, such as paste dentifrices, powder dentifrices, cream dentifrices, gel dentifrices, liquid dentifrices, pastes, and the like. In particular, paste dentifrices, powder dentifrices, cream dentifrices, or gel dentifrices are preferable.

The composition for oral use of the present invention can be produced, for example, by mixing diamond particles and silica particles (and other components, if necessary) with a base material that is pharmaceutically acceptable or hygienically acceptable in the oral cavity. Examples of such base materials include water, glycerol, ethylene glycol, diethylene glycol, polyethylene glycol, propylene glycol, polypropylene glycol, sorbitol, xylitol, lactitol, mannitol, ethanol, and the like.

The composition for oral use of the present invention may contain other components (optional components) that are usually incorporated into compositions for oral use.

Examples of surfactants that can be incorporated include nonionic surfactants, anionic surfactants, and amphoteric surfactants. Specific examples of nonionic surfactants include fatty acid esters, fatty acid alkanolamides, sorbitan fatty acid esters, fatty acid monoglycerides, polyglycerol fatty acid esters, polyoxyethylene alkyl phenyl ethers, alkyl glycosides, diethyl sebacate, polyoxyethylene hydrogenated castor oils, polyoxyethylene sorbitan fatty acid esters, and the like. Examples of anionic surfactants include alkyl sulfates (such as sodium lauryl sulfate), polyoxyethylene alkyl ether sulfates, alkyl sulfosuccinates, polyoxyethylene alkyl ether sulfosuccinates, N-acylamino acid salts, N-acyltaurine salts, alkyl ether carboxylates, alkyl phosphates, polyoxyethylene alkyl ether phosphates, fatty acid monoglyceride sulfates, alkyl sulfoacetates, and the like. Examples of amphoteric surfactants include alkyl dimethyl aminoacetate betaines, alkyl amidopropyldimethyl aminoacetate betaines, N-acyl-N-carboxymethyl-N-hydroxyethylethylenediamines, N-alkylaminoethylglycines, and the like. These surfactants can be used singly or in a combination of two or more. Such surfactants are typically incorporated in an amount of 0.1 to 10% by mass, based on the total mass of the composition.

Examples of thickeners that can be incorporated include cellulose derivatives such as carrageenan, carboxymethyl cellulose or salts thereof, hydroxypropyl cellulose, hydroxypropyl methylcellulose, hydroxyethyl cellulose, and crystalline cellulose-carmellose sodium; gums such as xanthan gum, tragacanth gum, karaya gum, gum arabic, and gellan gum; synthetic binders such as polyvinyl alcohol, sodium polyacrylate, carboxy vinyl polymer, and polyvinylpyrrolidone; inorganic binders such as thickening silica, silylated silica, aluminum silica gel, and veegum; sodium alginate, pectin, soybean polysaccharides, sodium chondroitin sulfate, sodium hyaluronate, and the like. These thickeners can be used singly or in a combination of two or more. Such thickeners are typically incorporated in an amount of 0.01 to 20% by mass.

Examples of flavoring agents that can be incorporated include menthol, carboxylic acid, anethole, eugenol, methyl salicylate, limonene, ocimene, n-decyl alcohol, citronellal, $\alpha$-terpineol, methyl acetate, citronellyl acetate, methyleugenol, cineol, linalool, ethyl linalool, thymol, spearmint oil, peppermint oil, lemon oil, orange oil, sage oil, rosemary oil, cinnamon oil, beefsteak plant oil, wintergreen oil, clove oil, eucalyptus oil, pimento oil, d-camphor, d-borneol, fennel oil, cinnamon oil, cinnamaldehyde, mint oil, vanillin, and the like. These flavoring agents can be used singly or in a combination of two or more. Such flavoring agents can be typically used in an amount of 0.01 to 1% by mass, based on the total mass of the composition.

Examples of sweetening agents that can be incorporated include saccharin sodium, acesulfame potassium, stevioside, stevia extract, neohesperidyl dihydrochalcone, glycyrrhizin, perillartine, thaumatin, asparatyl phenylalanyl methyl ester, and p-methoxycinnamic aldehyde, and the like. These sweetening agents can be used singly or in a combination of two or more. Such sweetening agents can be typically incorporated in an amount of 0.01 to 1% by mass, based on the total mass of the composition.

Examples of wetting agents that can be incorporated include sorbitol, ethylene glycol, propylene glycol, glycerol, 1,3-butylene glycol, polypropylene glycol, xylitol, maltitol, lactitol, Palatinit, polyethylene glycol, and the like. These wetting agents can be used singly or in a combination of two or more.

Examples of preservatives that can be incorporated include parabens such as methylparaben, ethylparaben, propylparaben, and butylparaben; sodium benzoate, phenoxyethanol, alkyldiaminoethylglycine hydrochloride, and the like. These preservatives can be used singly or in a combination of two or more.

Examples of colorants that can be incorporated include legal colors such as Blue No. 1, Yellow No. 4, Red No. 202, and Green No. 3; mineral-based pigments such as ultramarine blue, deep ultramarine blue, and Prussian blue; titanium oxide; and the like. These colorants can be used singly or in a combination of two or more.

Examples of pH adjusters that can be incorporated include citric acid, phosphoric acid, malic acid, pyrophosphoric acid, lactic acid, tartaric acid, glycerophosphoric acid, acetic acid, nitric acid, chemically acceptable salts thereof, sodium hydroxide, and the like. These pH adjusters can be used singly or in a combination of two or more so that the resulting composition has a pH of 4 to 8, and more preferably 5 to 7. Such pH adjusters can be incorporated, for example, in an amount of 0.01 to 2 wt.%.

The composition for oral use of the present invention may contain, as a medicinal ingredient, cationic disinfectants such as cetylpyridinium chloride, benzalkonium chloride, benzethonium chloride, and chlorhexidine hydrochloride; vitamin E such as dl-$\alpha$-tocopherol acetate, tocopherol succinate, and tocopherol nicotinate; amphoteric disinfectants such as dodecyldiaminoethylglycine; nonionic disinfectants such as triclosan and isopropylmethylphenol; enzymes such as dextranase, amylase, protease, mutanase, lysozyme, and lytic enzymes; fluorides such as sodium monofluorophosphate, sodium fluoride, and stannous fluoride; tranexamic acid, epsilon aminocaproic acid, aluminum chlorohydroxy allantoin, dihydrocholesterol, glycyrrhetinic acid, glycerophosphate, chlorophyll, sodium chloride, calpeptide, dipotassium glycyrrhizinate, allantoin, hinokitiol, potassium nitrate, and the like. These components can be used singly or in a combination of two or more.

There is no particular limitation on the container for holding the composition for oral use of the present invention. For example, a container made of glass, metal, plastic, a laminate material, or the like may be used. Further, the shape of the container is also not particularly limited. For example, a container such as a bottle, a cup, a pouch, or a tube can be used. Examples

The present invention is described below in more detail with reference to Examples but is not limited to these. The diamond particles used in the Examples below were purchased from Microdiamant, and the silica particles used in the Examples were purchased from J. M. Huber Corporation.

Preparation of the Composition for Oral Use

Compositions (pastes) for oral use in Examples 1 to 12 and Comparative Examples 1 to 4 were prepared according to the formulations shown in Tables 1 and 2 below. Specifically, starting materials other than diamond particles, abrasive silica particles, a flavoring agent, and foaming agents (sodium sulfosuccinate and sodium lauryl sulfate) were mixed. Diamond particles and abrasive silica particles were added, and the resulting mixture was stirred and homogenized. Further, the flavoring agent was added and the resulting mixture was stirred and homogenized. Lastly, the foaming agents were added and the resulting mixture was stirred and homogenized (this process also functioned as defoaming treatment). All numerals for the components in Tables 1 and 2 are by mass%.

Two types of commercially available precipitated silica products were used as silica particles. These were termed "silica particles a" and "silica particles b." The specifications of these commercially available silica products are as follows. Silica particles a have an average particle size (d50) of 3.5 to 5 $\mu$m, and an oil absorption (linseed oil absorption according to JIS K5101) of 30 to 70 (mL/100 g). Silica particles b have an average particle size (d50) of 6 to 12 $\mu$m, and an oil absorption (linseed oil absorption according to JIS K5101) of 90 to 135 (mL/100 g).

The diamond particles used had an average particle size (d50) of 2.4 to 3 $\mu$m (about 2.8 $\mu$m), a d10 of 1.8 $\mu$m or more, and a d99 of 4.5 $\mu$m or less.

The particle size of silica particles (d50) is determined by using a Malvern Mastersizer based on the principles of Fraunhofer diffraction using a low-power He/Ne laser. The cumulative diameter of the diamond particles is determined by using a Multisizer 3 Coulter Counter (produced by Beckman Coulter, Inc.)

Evaluation of Compositions for Oral Use

The obtained compositions for oral use were evaluated for cleaning ability and smoothing in the following manner. Table 2 shows the results.

Method for Evaluating Cleaning Ability

A bovine enamel tooth fragment was cut out to a size of 3 mm in length x 3 mm in width using an automatic precision cutter. Two pieces of the fragment were embedded per block using a dental polymer resin (Orthofast resin, produced by GC Corporation). After drying, the surface of the tooth fragment was mirror-polished with an automatic polishing device and polishing paper, washed with ion-exchanged water, and dried. To determine the initial color difference, L*, a*, and b* values were measured in a 1.8-mm-diameter measurement area using a colorimeter (CR-241, produced by Konica Minolta Sensing, Inc.) (The L, a, and b values thus obtained were termed $L^0$, $a^0$, and $b^0$, respectively.)

Further, the surface of the tooth fragment subjected to the initial color difference measurement was etched by sequentially immersing 0.2M hydrochloric acid, aqueous saturated sodium carbonate solution, distilled water, 1% (v/v) phytic acid, and distilled water in this order.

Subsequently, stains were applied to the surface of the etched tooth fragment in the following manner. The staining fluid to be used was prepared by placing four commercially available tea bags into about 1.2 L of hot water, boiling the water for 10 minutes, cooling the resulting black tea solution to room temperature, and adding 3.4 g of commercially available instant coffee and 2.5 g of type II porcine stomach mucin (produced by Sigma-Aldrich Co. LLC).

The tooth fragment was placed on a stain application machine. The staining fluid was added and the machine was rotated to continue a stain application treatment for 5 to 6 days. The staining fluid was replaced with new staining fluid once a day. The tooth fragment was then treated with a treatment liquid, which was prepared by adding 1 g of ferric chloride to the staining fluid, for 1 or 2 days until the L* value became 30 or less. After the tooth fragment was washed with ion-exchanged water and dried, the color difference was measured ($L^1$, $a^1$, $b^1$).

The stained tooth fragment was set in an ISO-standard brushing machine. The stained tooth fragment was brushed for 3,000 reciprocal strokes with a load of 150 g at a rate of 150 rpm in a 3-fold dilution (60 ml) of each of the compositions for oral use obtained in the Examples and Comparative Examples. After drying, the color difference was measured ($L^2$, $a^2$, $b^2$). The stain removal ability ($\Delta E$) was calculated according to the following formula (rounded off to the nearest whole number) and evaluated. Table 2 shows $\Delta E$ as the value reflecting cleaning ability.

$$\Delta E = (\Delta E^1 / \Delta E^0) \times 100$$

provided, however, that

$$\Delta E^1 = \{ (L^2 - L^1)^2 + (a^2 - a^1)^2 + (b^2 - b^1)^2 \}^{1/2}$$

$$\Delta E^0 = \{ (L^2 - L^0)^2 + (a^2 - a^0)^2 + (b^2 - b^0)^2 \}^{1/2}$$

(That is, $\Delta E^1$ is equal to the square root of the sum of the square of ($L^2$-$L^1$), the square of ($a^2$-$a^1$), and the square of ($b^2$-$b^1$), and $\Delta E^0$ is equal to the square root of the sum of the square of ($L^2$-$L^0$), the square of ($a^2$-$a^0$), and the square of ($b^2$-$b^0$).)

Evaluation of Smoothness

A bovine enamel tooth fragment was cut out to a size of 3 mm in length x 6 mm in width using an automatic precision cutter. One piece of the fragment was embedded in a dental polymer resin (Orthofast resin, produced by GC Corporation) per block. After drying, the surface of the tooth fragment was mirror-polished with an automatic polishing device and polishing paper.

The surface of the tooth fragment was etched by sequentially immersing 0.2M hydrochloric acid, distilled water, aqueous saturated sodium carbonate solution, distilled water, and 1% (v/v) phytic acid in this order.

After tape (Scotch brand tape, 10 mm; Sumitomo 3M) was applied to half of the tooth surface to protect the surface, the tooth fragment was set in an ISO-standard brushing machine. The tooth fragment was brushed for 3,000 reciprocal strokes in a 3-fold dilution (60 mL) of the compositions for oral use of the Examples and Comparative Examples with a load of 150 g at a rate of 150 rpm.

After brushing, the arithmetic-average roughness (Ra) per unit area of 500 $\mu$m x 500 $\mu$m was measured with a shape-measuring laser microscope (VK-8700, produced by Keyence Corporation) and evaluated. The Ra of the surface protected with the tape was termed Ra before testing. The smoothness was calculated according to the following formula.

$$\text{Smoothness} = \{(\text{Ra before testing} - \text{Ra after testing})/\text{Ra before testing}\} \times 100$$

Table 1

| Concentrated glycerin | 10 |
|---|---|
| Polyethylene glycol 400 | 3 |
| Sorbitol solution (70%) | 15 |
| Polyoxyethylene hydrogenated castor oil | 0.5 |
| Saccharin sodium | 0.2 |
| Stevia extract | 0.1 |
| Silica particles a | See Table 2. |
| Silica particles b | See Table 2. |
| Diamond particles | See Table 2. |
| Sodium carboxymethyl cellulose | 0.5 |
| Xanthan gum | 0.5 |
| Flavoring agent | 0.1 |
| Disodium polyoxyethylenealkyl ($C_{12-14}$) sulfosuccinate | 4 |
| Sodium lauryl sulfate | 1 |
| Purified water | Balance |
| Total | 100 |

Table 2

| | Comparative Example 1 | Comparative Example 2 | Comparative Example 3 | Example 1 | Example 2 | Example 3 | Example 4 | Example 5 |
|---|---|---|---|---|---|---|---|---|
| Diamond particles | | | 0.2 | 0.2 | 0.2 | 0.2 | 0.2 | 0.2 |
| Silica particles a | | 5 | | 3 | 5 | | | |
| Silica particles b | 21.5 | 15 | | | | 5 | 15 | 21.5 |
| Cleaning ability | 5.4 | 8.6 | 3.3 | 8.5 | 10.2 | 6.7 | 8.3 | 11.2 |
| Smoothness | 11 | 13 | 62 | 38 | 36 | 42 | 36 | 51 |
| | Example 6 | Example 7 | Example 8 | Example 9 | Comparative Example 4 | Example 10 | Example 11 | Example 12 |
| Diamond particles | 0.2 | 0.2 | 0.2 | 0.2 | | 0.1 | 0.05 | 0.01 |
| Silica particles a | 3 | 3 | 5 | 5 | 7 | 7 | 7 | 7 |
| Silica particles b | 5 | 15 | 15 | 21.5 | 18 | 18 | 18 | 18 |
| Cleaning ability | 6.0 | 9.7 | 10.2 | 11.1 | 10.0 | 12.0 | 12.4 | 11.4 |
| Smoothness | 51 | 39 | 49 | 45 | 23 | 43 | 42 | 41 |

The results show that the composition for oral use of the present invention comprising diamond particles and silica particles has excellent cleaning ability and smoothing.

Formulation Examples are shown below. The following diamond particles and silica particles were used in the Formulation Examples. The amount (%) herein indicates mass%.

Diamond Particles and Silica Particles Used

Diamond particles A: average particle size: 2.8 $\mu$m (d50), at least 1.8 $\mu$m (d10), not more than 4.5 $\mu$m (d99).
Diamond particles B: average particle size: 0.6 $\mu$m (d50), at least 0.1 $\mu$m (d10), not more than 2 $\mu$m (d99).
Diamond particles C: average particle size: 4.9 $\mu$m (d50), at least 1.8 $\mu$m (d10), not more than 7 $\mu$m (d99).
Diamond particles D: average particle size: 2 $\mu$m (d50), at least 1 $\mu$m (d10), not more than 4.5 $\mu$m (d99).
Diamond particles E: average particle size: 2.1 $\mu$m (d50), at least 1.3 $\mu$m (d10), not more than 4.5 $\mu$m (d99).
Diamond particles F: average particle size: 3 $\mu$m (d50), 1.8 $\mu$m (d10), not more than 5 $\mu$m (d99).
Silica particles A: average particle size: 4.5 $\mu$m (d50), oil absorption: 52 (mL/100 g).
Silica particles B: average particle size: 10.5 $\mu$m (d50), oil absorption: 118 (mL/100 g).
Silica particles C: average particle size: 3.2 $\mu$m (d50), oil absorption: 76 (mL/100 g).
Silica particles D: average particle size: 14.5 $\mu$m (d50), oil absorption: 146 (mL/100 g).
Silica particles E: average particle size: 5.3 $\mu$m (d50), oil absorption: 78 (mL/100 g).
Silica particles F: average particle size 6.3 $\mu$m (d50), oil absorption: 139 (mL/100 g).
Silica particles G: average particle size: 5.1 $\mu$m (d50), oil absorption: 28 (mL/100 g).
Silica particles H: average particle size: 6.3 $\mu$m (d50), oil absorption: 99 (mL/100 g).
Silica particles I: average particle size: 3.4 $\mu$m (d50), oil absorption: 30 (mL/100 g).
Silica particles J: average particle size: 13.3 $\mu$m (d50), oil absorption: 94 (mL/100 g).

Formulation Example 1: Paste dentifrice

| Component | Amount (%) |
| --- | --- |
| Diamond particle A | 0.2 |
| Silica particle A | 5.0 |
| Silica particle B | 15.0 |
| Thickening silica | 5.0 |
| Sorbit solution (70%) | 40.0 |
| Xanthan gum | 0.6 |
| Sodium pyrophosphate | 3.0 |
| Sodium phosphate | 0.5 |
| Disodium hydrogen phosphate | 0.2 |
| Sodium fluoride | 0.2 |
| Triclosan | 0.05 |
| Titanium oxide | 0.5 |
| Sodium lauryl sulfate | 1.0 |
| Flavoring agent | 1.0 |
| Saccharin sodium | 0.2 |
| Water | Balance |
| Total | 100.0 |

Formulation Example 2: Gel dentifrice

| Component | Amount (%) |
| --- | --- |
| Diamond particle B | 0.2 |
| Silica particle C | 1.0 |
| Silica particle D | 25.0 |
| Calcium hydrogen phosphate | 5.0 |
| Crystalline cellulose | 5.0 |
| Xanthan gum | 0.6 |
| Sodium polyacrylate | 0.3 |

(continued)

| Component | Amount (%) |
|---|---|
| Sodium monofluorophosphate | 0.7 |
| Potassium nitrate | 5.0 |
| Tocopherol nicotinate | 0.2 |
| Glycerol monostearate | 1.2 |
| Flavoring agent | 1.0 |
| Saccharin sodium | 0.2 |
| Reducing palatinose | 5.0 |
| Water | Balance |
| Total | 100.0 |

Formulation Example 3: Gel dentifrice

| Component | Amount (%) |
|---|---|
| Diamond particle C | 0.2 |
| Silica particle E | 10.0 |
| Silica particle F | 5.0 |
| Calcium carbonate | 10.0 |
| Glycerol | 20.0 |
| Xanthan gum | 0.7 |
| Sodium carboxymethyl cellulose | 0.5 |
| Sodium polyacrylate | 0.4 |
| Sodium monofluorophosphate | 0.7 |
| Tocopherol acetate | 0.1 |
| Lauroylsarcosine sodium | 0.1 |
| Sodium lauryl sulfate | 1.0 |
| Methylparaben | 0.1 |
| Flavoring agent | 1.0 |
| Saccharin sodium | 0.2 |
| Water | Balance |
| Total | 100.0 |

Formulation Example 4: Gel dentifrice

| Component | Amount (%) |
|---|---|
| Diamond particle B | 0.2 |
| Silica particle G | 1.0 |
| Silica particle H | 3.0 |
| Aluminum hydroxide | 20.0 |
| Thickening silica | 10.0 |
| Glycerol | 20.0 |
| Sodium carboxymethyl cellulose | 0.7 |
| Sodium monofluorophosphate | 0.7 |
| Dipotassium glycyrrhizinate | 0.02 |
| Polyoxyethylene(60) hydrogenated castor oil | 1.0 |
| Alkyl ($C_{8-16}$) glucoside | 2.0 |
| Flavoring agent | 1.0 |
| Saccharin sodium | 0.05 |
| L-menthol | 0.5 |
| Water | Balance |

(continued)

| Component | Amount (%) |
|---|---|
| Total | 100.0 |

Formulation Example 5: Paste dentifrice

| Component | Amount (%) |
|---|---|
| Diamond particle F | 0.2 |
| Silica particle I | 10.0 |
| Silica particle J | 25.0 |
| Glycerol | 20.0 |
| Hydroxyethylcellulose | 0.7 |
| Carrageenan | 0.3 |
| Sodium alginate | 0.7 |
| Sodium monofluorophosphate | 0.7 |
| Dipotassium glycyrrhizinate | 0.02 |
| Polyoxyethylene(60) hydrogenated castor oil | 1.0 |
| Alkyl ($C_{8-16}$) glucoside | 1.5 |
| Flavoring agent | 1.0 |
| Saccharin sodium | 0.05 |
| Reduced palatinose | 10.0 |
| Water | Balance |
| Total | 100.0 |

Formulation Example 6: Liquid dentifrice

| Component | Amount (%) |
|---|---|
| Diamond particle C | 0.2 |
| Silica particle A | 1.0 |
| Polyethylene powder | 3.0 |
| Sorbit solution (70%) | 20.0 |
| Glycerol | 8.0 |
| Polyethylene glycol 200 | 3.0 |
| Xanthan gum | 0.1 |
| Sodium carboxymethyl cellulose | 0.1 |
| Sodium hydroxide | 1.0 |
| Erythritol | 5.0 |
| Tocopherol acetate | 0.1 |
| Triclosan | 0.1 |
| Propylene glycol monostearate | 1.0 |
| Titanium oxide | 0.5 |
| Flavoring agent | 1.0 |
| Saccharin sodium | 0.2 |
| L-menthol | 0.5 |
| Water | Balance |
| Total | 100.0 |

Formulation Example 7: Paste dentifrice

| Component | Amount (%) |
|---|---|
| Diamond particle E | 0.2 |

(continued)

| Component | Amount (%) |
|---|---|
| Silica particle B | 30.0 |
| Silylated silica | 2.0 |
| Sorbit solution (70%) | 25.0 |
| Polyethylene glycol 600 | 3.0 |
| Xanthan gum | 0.3 |
| Sodium carboxymethyl cellulose | 0.2 |
| Sodium hydroxide | 1.0 |
| Phytic acid | 0.5 |
| Tocopherol acetate | 0.1 |
| Triclosan | 0.1 |
| Sodium lauryl sulfate | 1.0 |
| Titanium oxide | 0.5 |
| Flavoring agent | 1.0 |
| Saccharin sodium | 0.2 |
| L-menthol | 0.5 |
| Water | Balance |
| Total | 100.0 |

### Formulation Example 8: Paste dentifrice

| Component | Amount (%) |
|---|---|
| Diamond particle C | 0.01 |
| Silica particle C | 5.0 |
| Silica particle F | 15.0 |
| Calcium hydrogen phosphate for Dentifrice | 5.0 |
| Glycerol | 8.0 |
| Polyethylene glycol 1000 | 3.0 |
| Sodium carboxymethyl cellulose | 0.7 |
| Magnesium phosphate | 0.5 |
| Hydroxyapatite | 2.0 |
| Lauroylsarcosine sodium | 1.0 |
| Zeolite | 1.5 |
| Glycerol monostearate | 1.2 |
| Flavoring agent | 1.0 |
| Saccharin sodium | 0.2 |
| Water | Balance |
| Total | 100.0 |

### Formulation Example 9: Paste dentifrice

| Component | Amount (%) |
|---|---|
| Diamond particle A | 0.01 |
| Silica particle E | 1.0 |
| Silica particle H | 25.0 |
| Powdered cellulose | 5.0 |
| Sorbit solution (70%) | 20.0 |
| Polyethylene glycol 1500 | 3.0 |
| Carrageenan | 0.7 |
| Glycerol fatty acid ester | 1.0 |

(continued)

| Component | Amount (%) |
|---|---|
| Potassium hydroxide solution | 1.0 |
| DL-malic acid | 0.5 |
| Sodium fluoride | 0.2 |
| Sodium lauryl sulfate | 1.0 |
| Titanium oxide | 0.5 |
| Flavoring agent | 1.0 |
| Saccharin sodium | 0.2 |
| L-menthol | 0.5 |
| Water | Balance |
| Total | 100.0 |

Formulation Example 10: Paste dentifrice

| Component | Amount (%) |
|---|---|
| Diamond particle D | 0.01 |
| Silica particle G | 10.0 |
| Silica particle J | 5.0 |
| Sorbit solution (70%) | 20.0 |
| Polyethylene glycol 2000 | 3.0 |
| Sodium fluoride | 0.2 |
| Sodium lauryl sulfate | 1.0 |
| Titanium oxide | 0.5 |
| Mica titanium | 0.5 |
| Flavoring agent | 1.0 |
| Saccharin sodium | 0.2 |
| Peppermint oil | 0.5 |
| Water | Balance |
| Total | 100.0 |

Formulation Example 11: Liquid dentifrice

| Component | Amount (%) |
|---|---|
| Diamond particle A | 0.01 |
| Silica particle I | 1.0 |
| Silica particle D | 3.0 |
| Tricalcium phosphate | 5.0 |
| Calcium hydrogen phosphate | 10.0 |
| Sorbit solution (70%) | 20.0 |
| Ethanol | 5.0 |
| Polyethylene glycol 400 | 3.0 |
| Magnesium phosphate | 0.5 |
| Sodium carboxymethyl cellulose | 0.7 |
| Sodium monofluorophosphate | 0.7 |
| Zeolite | 0.5 |
| $\varepsilon$-aminocaproic acid | 0.1 |
| Sodium lauryl sulfate | 1.0 |
| Paraben | 0.1 |
| Flavoring agent | 1.0 |
| Xylitol | 0.2 |

(continued)

| Component | Amount (%) |
|---|---|
| L-menthol | 0.5 |
| Water | Balance |
| Total | 100.0 |

**Formulation Example 12: Paste dentifrice**

| Component | Amount (%) |
|---|---|
| Diamond particle B | 0.01 |
| Silica particle A | 10.0 |
| Silica particle J | 25.0 |
| Thickening silica | 1.0 |
| Sorbit solution (70%) | 20.0 |
| Glycerol | 8.0 |
| Propylene glycol | 5.0 |
| Polyethylene glycol 4000 | 1.0 |
| Sodium carboxymethyl cellulose | 0.7 |
| Sodium fluoride | 0.2 |
| Isopropylmethyl phenol | 0.05 |
| Sodium lauryl sulfate | 1.0 |
| Disodium polyoxyethylene (2 moles) alkyl ($C_{12-14}$) sulfosuccinate | 1.0 |
| Polyoxyethylene(60) hydrogenated castor oil | 1.0 |
| Titanium oxide | 0.5 |
| Flavoring agent | 1.0 |
| Saccharin sodium | 0.2 |
| Stevia extract | 0.1 |
| Water | Balance |
| Total | 100.0 |

**Formulation Example 13: Liquid dentifrice**

| Component | Amount (%) |
|---|---|
| Diamond particle B | 0.01 |
| Silica particle H | 1.0 |
| Thickening silica | 3.0 |
| Sorbit solution (70%) | 20.0 |
| Propylene glycol | 5.0 |
| Xanthan gum | 0.7 |
| Sodium hydrogen carbonate | 0.5 |
| Sodium monofluorophosphate | 0.7 |
| Lauroylsarcosine sodium | 1.0 |
| Dextranase | 0.5 |
| Sodium lauryl sulfate | 1.0 |
| Paraben | 0.1 |
| DL-alanine | 0.5 |
| Flavoring agent | 1.0 |
| I-menthol | 0.5 |
| Water | Balance |
| Total | 100.0 |

Formulation Example 14: Paste dentifrice

| Component | Amount (%) |
|---|---|
| Diamond particle D | 0.01 |
| Silica particle F | 30.0 |
| Aluminum hydroxide | 8.0 |
| Thickening silica | 3.0 |
| Sorbit solution (70%) | 20.0 |
| Propylene glycol | 8.0 |
| Xanthan gum | 0.7 |
| 2-Alkyl-N-hydroxyethyl imidazolinium betaine | 0.5 |
| Sodium hydrogen carbonate | 0.5 |
| Sodium monofluorophosphate | 0.7 |
| Lauroylsarcosine sodium | 1.0 |
| Dextranase | 0.5 |
| Sodium lauryl sulfate | 1.0 |
| Paraben | 0.1 |
| Titanium oxide | 0.5 |
| Flavoring agent | 1.0 |
| Saccharin sodium | 0.2 |
| L-menthol | 0.5 |
| Water | Balance |
| Total | 100.0 |

Formulation Example 15: Paste dentifrice

| Component | Amount (%) |
|---|---|
| Diamond particle C | 0.5 |
| Silica particle E | 5.0 |
| Silica particle D | 15.0 |
| Gel silica | 5.0 |
| Sorbit solution (70%) | 20.0 |
| Xanthan gum | 0.7 |
| Sodium polyphosphate | 0.7 |
| Sodium alginate | 0.7 |
| Sodium fluoride | 0.2 |
| Lauroylsarcosine sodium | 1.0 |
| Hydroxyethylcellulose dimethyldiallyl ammonium chloride | 0.2 |
| Benzalkonium chloride | 0.1 |
| Sodium lauryl sulfate | 0.5 |
| Polyoxyethylene(60) hydrogenated castor oil | 1.0 |
| Titanium oxide | 0.5 |
| Flavoring agent | 1.0 |
| Xylitol | 0.2 |
| L-menthol | 0.5 |
| Water | Balance |
| Total | 100.0 |

### Formulation Example 16: Paste dentifrice

| Component | Amount (%) |
|---|---|
| Diamond particle E | 0.5 |
| Silica particle G | 1.0 |
| Silica particle D | 25.0 |
| Thickening fumed silica | 5.0 |
| Sorbit solution (70%) | 20.0 |
| Propylene glycol | 8.0 |
| Polyethylene glycol 6000 | 3.0 |
| Xanthan gum | 0.7 |
| Sodium fluoride | 0.2 |
| Isopropylmethyl phenol | 0.05 |
| ε-Aminocaproic acid | 0.1 |
| Sodium lauryl sulfate | 1.0 |
| Titanium oxide | 0.5 |
| Flavoring agent | 1.0 |
| Saccharin sodium | 0.2 |
| L-menthol | 0.5 |
| Water | Balance |
| Total | 100.0 |

### Formulation Example 17: Paste dentifrice

| Component | Amount (%) |
|---|---|
| Diamond particle B | 0.5 |
| Silica particle E | 10.0 |
| Silica particle B | 5.0 |
| Calcium carbonate | 15.0 |
| Sorbit solution (70%) | 20.0 |
| Glycerol | 5.0 |
| Sodium carboxymethyl cellulose | 0.7 |
| Polyoxyethylene sorbitan monostearate | 0.5 |
| Sodium hydroxide | 0.5 |
| Sodium chloride | 15.0 |
| Sodium monofluorophosphate | 0.7 |
| β-Glycyrrhetic acid | 0.02 |
| Sodium lauryl sulfate | 1.0 |
| Titanium oxide | 0.5 |
| Flavoring agent | 1.0 |
| Saccharin sodium | 0.2 |
| Water | Balance |
| Total | 100.0 |

### Formulation Example 18: Paste dentifrice

| Component | Amount (%) |
|---|---|
| Diamond particle C | 0.5 |
| Silica particle G | 1.0 |
| Silica particle B | 3.0 |
| Sorbit solution (70%) | 20.0 |
| Carrageenan | 0.7 |

(continued)

| Component | Amount (%) |
|---|---|
| Sodium carboxymethyl cellulose | 0.7 |
| Sodium N-lauroyl-L-glutamate | 0.5 |
| Sodium hydrogen carbonate | 0.01 |
| Sodium chloride | 10.0 |
| Sodium monofluorophosphate | 0.7 |
| Benzethonium chloride | 0.1 |
| Sodium lauryl sulfate | 1.0 |
| Titanium oxide | 0.5 |
| Flavoring agent | 1.0 |
| Saccharin sodium | 0.2 |
| Water | Balance |
| Total | 100.0 |

Formulation Example 19: Paste dentifrice

| Component | Amount (%) |
|---|---|
| Diamond particle F | 0.5 |
| Silica particle I | 10.0 |
| Silica particle F | 25.0 |
| Gel silica | 10.0 |
| Calcium carbonate | 5.0 |
| Zinc oxide | 1.0 |
| Sorbit solution (70%) | 20.0 |
| Polyethylene glycol 11000 | 3.0 |
| Sodium carboxymethyl cellulose | 0.7 |
| Sodium monofluorophosphate | 0.7 |
| Benzethonium chloride | 0.1 |
| Sodium lauryl sulfate | 1.0 |
| Flavoring agent | 1.0 |
| Saccharin sodium | 0.2 |
| Water | Balance |
| Total | 100.0 |

Formulation Example 20: Paste dentifrice

| Component | Amount (%) |
|---|---|
| Diamond particle A | 0.5 |
| Silica particle H | 1.0 |
| Zinc oxide | 1.0 |
| Sorbit solution (70%) | 20.0 |
| Glycerol | 5.0 |
| Polyethylene glycol 20000 | 3.0 |
| Polyethylene powder | 0.5 |
| Xanthan gum | 0.7 |
| Sodium carboxymethyl cellulose | 0.7 |
| Cocamide propylbetaine | 0.5 |
| Sodium hydroxide | 0.5 |
| Sodium tripolyphosphate | 0.3 |
| Sodium pyrophosphate | 0.2 |

(continued)

| Component | Amount (%) |
| --- | --- |
| Sodium monofluorophosphate | 0.7 |
| Sodium lauryl sulfate | 1.0 |
| Titanium oxide | 0.5 |
| Flavoring agent | 1.0 |
| Saccharin sodium | 0.2 |
| Water | Balance |
| Total | 100.0 |

Formulation Example 21: Paste dentifrice

| Component | Amount (%) |
| --- | --- |
| Diamond particle A | 0.5 |
| Silica particle I | 30.0 |
| Sorbit solution (70%) | 20.0 |
| Propylene glycol | 3.0 |
| Carrageenan | 0.7 |
| Sodium carboxymethyl cellulose | 0.7 |
| Sodium hydroxide | 0.5 |
| Methyl vinyl ether-maleic acid copolymer | 0.5 |
| Sodium fluoride | 0.2 |
| Triclosan | 0.1 |
| Sodium lauryl sulfate | 1.0 |
| Titanium oxide | 0.5 |
| Flavoring agent | 1.0 |
| Saccharin sodium | 0.2 |
| Water | Balance |
| Total | 100.0 |

Formulation Example 22: Paste dentifrice

| Component | Amount (%) |
| --- | --- |
| Diamond particle A | 0.05 |
| Silica particle E | 3.0 |
| Silica particle D | 18.0 |
| Gel silica | 5.0 |
| Sorbit solution (70%) | 10.0 |
| Glycerol | 5.0 |
| Polyethylene glycol 300 | 5.0 |
| Xanthan gum | 0.7 |
| Sodium carboxymethyl cellulose | 0.7 |
| Sodium hydroxide | 0.5 |
| Sodium metaphosphate | 0.5 |
| Cocamide propylbetaine | 0.3 |
| Carbomer 956 | 0.3 |
| Poloxamer 407 | 0.2 |
| Sodium monofluorophosphate | 0.7 |
| Sodium lauryl sulfate | 1.0 |
| Titanium oxide | 0.5 |
| Flavoring agent | 1.0 |

(continued)

| Component | Amount (%) |
|---|---|
| Saccharin sodium | 0.2 |
| Water | Balance |
| Total | 100.0 |

### Formulation Example 23: Paste dentifrice

| Component | Amount (%) |
|---|---|
| Diamond particle B | 0.15 |
| Silica particle I | 3.0 |
| Silica particle H | 12.0 |
| Sorbit solution (70%) | 10.0 |
| Polyethylene glycol 1540 | 5.0 |
| Xanthan gum | 1.0 |
| Anhydrous sodium monohydrogen phosphate | 0.5 |
| Sodium benzoate | 0.5 |
| Sodium sulfate | 0.5 |
| Sodium fluoride | 0.2 |
| Polyoxyethylene lauryl ether sodium sulfate | 1.0 |
| Titanium oxide | 0.5 |
| Mica | 0.2 |
| Flavoring agent | 1.0 |
| Saccharin sodium | 0.2 |
| Water | Balance |
| Total | 100.0 |

### Formulation Example 24: Gel dentifrice

| Component | Amount (%) |
|---|---|
| Diamond particle C | 0.25 |
| Silica particle C | 2.0 |
| Silica particle B | 8.0 |
| Sorbit solution (70%) | 10.0 |
| Glycerol | 5.0 |
| Polyethylene glycol 400 | 5.0 |
| Sodium carboxymethyl cellulose | 0.7 |
| Anhydrous sodium monohydrogen phosphate | 1.0 |
| Lecithin | 0.5 |
| Methyl vinyl ether-maleic acid copolymer | 0.5 |
| Sodium fluoride | 0.2 |
| Sodium lauryl sulfate | 1.0 |
| Mica | 0.2 |
| Flavoring agent | 1.0 |
| Saccharin sodium | 0.2 |
| Limonene | 0.5 |
| Water | Balance |
| Total | 100.0 |

Formulation Example 25: Paste dentifrice

| Component | Amount (%) |
|---|---|
| Diamond particle C | 0.3 |
| Silica particle G | 7.0 |
| Silica particle F | 15.0 |
| Glycerol | 5.0 |
| Propylene glycol | 5.0 |
| Polyethylene glycol 400 | 5.0 |
| Xanthan gum | 0.7 |
| Sodium carboxymethyl cellulose | 0.7 |
| Calcium peroxide | 1.0 |
| Sodium hydrogen carbonate | 0.5 |
| Sodium hydroxide | 0.5 |
| Poloxamer 407 | 0.3 |
| Potassium pyrophosphate | 0.2 |
| Sodium monofluorophosphate | 0.7 |
| Sodium lauryl sulfate | 1.0 |
| Titanium oxide | 0.5 |
| Flavoring agent | 1.0 |
| Saccharin sodium | 0.2 |
| Water | Balance |
| Total | 100.0 |


Formulation Example 26: Paste dentifrice

| Component | Amount (%) |
|---|---|
| Diamond particle B | 0.4 |
| Silica particle C | 8.0 |
| Silica particle J | 7.0 |
| Glycerol | 5.0 |
| Polyethylene glycol 4000 | 5.0 |
| Carrageenan | 0.7 |
| Polyvinylpyrrolidone | 0.5 |
| Sodium polyphosphate | 0.7 |
| Hydroxyethylcellulose dimethyldiallyl ammonium chloride | 0.2 |
| Sodium monofluorophosphate | 0.7 |
| Sodium lauryl sulfate | 1.0 |
| Polyoxyethylene(60) hydrogenated castor oil | 0.5 |
| Titanium oxide | 0.5 |
| Flavoring agent | 1.0 |
| Saccharin sodium | 0.2 |
| Water | Balance |
| Total | 100.0 |


Formulation Example 27: Paste dentifrice

| Component | Amount (%) |
|---|---|
| Diamond particle A | 0.02 |
| Silica particle A | 6.0 |
| Silica particle H | 20.0 |
| Glycerol | 5.0 |
| Polyethylene glycol 4000 | 5.0 |

(continued)

| Component | Amount (%) |
|---|---|
| Carrageenan | 0.7 |
| Polyvinylpyrrolidones | 0.5 |
| Sodium polyphosphate | 0.7 |
| Sodium monofluorophosphate | 0.7 |
| Sodium lauryl sulfate | 1.0 |
| Palm oil fatty acid amide propyl betaine | 1.0 |
| Polyoxyethylene(60) hydrogenated castor oil | 0.5 |
| Polyoxyethylene stearyl ether | 0.5 |
| Titanium oxide | 0.5 |
| Flavoring agent | 1.0 |
| Saccharin sodium | 0.2 |
| Anhydrous caffeine | 0.1 |
| Water | Balance |
| Total | 100.0 |

Formulation Example 28: Paste dentifrice

| Component | Amount (%) |
|---|---|
| Diamond particle A | 0.4 |
| Silica particle I | 4.0 |
| Silica particle A | 19.0 |
| Calcium carbonate | 5.0 |
| Glycerol | 5.0 |
| Propylene glycol | 5.0 |
| Polyethylene glycol 1540 | 5.0 |
| Powdered agar | 1.0 |
| Sodium carboxymethyl cellulose | 0.7 |
| Hydrogen peroxide | 0.5 |
| Sodium monofluorophosphate | 0.7 |
| Benzethonium chloride | 0.5 |
| Sodium N-stearoyl-N-methyltaurine | 0.5 |
| Sodium lauryl sulfate | 1.0 |
| Titanium oxide | 0.5 |
| Flavoring agent | 1.0 |
| Saccharin sodium | 0.2 |
| Water | Balance |
| Total | 100.0 |

**Claims**

1.  A composition for oral use comprising diamond particles and silica particles.

2.  The composition for oral use according to claim 1,
    wherein the diamond particles have an average particle size (d50) of 0.5 to 5 $\mu$m.

3.  The composition for oral use according to claim 1 or 2, wherein the silica particles have an average particle size (d50) of 0.5 to 20 $\mu$m.

4.  The composition for oral use according to any one of claims 1 to 3, wherein the diamond particles are present in an amount of 0.01 to 0.5 mass%.

5. The composition for oral use according to any one of claims 1 to 4, wherein the silica particles comprise a combination of first silica particles and second silica particles, the first silica particles having an average particle size (d50) of 3 to 5.5 $\mu$m and an oil absorption of 20 to 80 (mL/100 g), and second silica particles having an average particle size (d50) of 6 to 15 $\mu$m and an oil absorption of 90 to 150 (mL/100 g).

6. The composition for oral use according to any one of claims 1 to 5, wherein the silica particles are present in an amount of 1 to 35 parts by mass per 0.01 to 0.5 parts by mass of the diamond particles.

**EP 3 006 012 A1**

## INTERNATIONAL SEARCH REPORT

| International application No. |
|---|
| PCT/JP2014/064933 |

A. CLASSIFICATION OF SUBJECT MATTER
*A61K8/19*(2006.01)i, *A61K8/25*(2006.01)i, *A61Q11/00*(2006.01)i

According to International Patent Classification (IPC) or to both national classification and IPC

B. FIELDS SEARCHED

Minimum documentation searched (classification system followed by classification symbols)
A61K8/19, A61K8/25, A61Q11/00

Documentation searched other than minimum documentation to the extent that such documents are included in the fields searched
Jitsuyo Shinan Koho          1922–1996    Jitsuyo Shinan Toroku Koho    1996–2014
Kokai Jitsuyo Shinan Koho    1971–2014    Toroku Jitsuyo Shinan Koho    1994–2014

Electronic data base consulted during the international search (name of data base and, where practicable, search terms used)
Thomson Innovation

C. DOCUMENTS CONSIDERED TO BE RELEVANT

| Category* | Citation of document, with indication, where appropriate, of the relevant passages | Relevant to claim No. |
|---|---|---|
| X | JP 2007-091636 A  (GC Corp.), 12 April 2007 (12.04.2007), claims; paragraphs [0011] to [0013]; examples 1, 3, 4 (Family: none) | 1,2 |
| X | US 2009/0130031 A1  (Michael HERMAN), 21 May 2009 (21.05.2009), abstract; claims; example 5 & US 2009/0130627 A1 | 1-4,6 |
| Y | | 5,6 |
| X | US 2010/0254915 A1  (Lisa Marie KAO), 07 October 2010 (07.10.2010), abstract; claims; examples; paragraphs [0028] to [0033] (Family: none) | 1-4,6 |
| Y | | 5,6 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 08 August, 2014 (08.08.14) | 19 August, 2014 (19.08.14) |

| Name and mailing address of the ISA/ Japanese Patent Office | Authorized officer |
|---|---|
| Facsimile No. | Telephone No. |

Form PCT/ISA/210 (second sheet) (July 2009)

**23**

**INTERNATIONAL SEARCH REPORT**

International application No.

PCT/JP2014/064933

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-238321 A (Kao Corp.), 26 August 2004 (26.08.2004), claims; paragraphs [0008] to [0010], [0015] to [0017]; examples (Family: none) | 5,6 |
| A | HUBER DENTAL SILICAS ZEODENT, [online], 2011. 03, [retrieval date 08 August 2014 (08.08. 2014)], Internet, <URL:http://www. hubermaterials.com/userfiles/files/PFDocs/ Zeodent%20High%20Performing%20Dental%20Silicas %20%28European%20Version%29.pdf> | 1-6 |
| A | SIDENT 9, [online], 2013.03, [retrieval date 08 August 2014 (08.08.2014)], Internet, <URL:http: //www.sipernat.com/product/sipernat/Documents/ SIDENT-9-EN.pdf> | 1-6 |
| A | SIDENT 22S, [online], 2012.02, [retrieval date 08 August 2014 (08.08.2014)], Internet, <URL: http://www.sipernat.com/product/sipernat/ Documents/SIDENT-22-S-EN.pdf> | 1-6 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013120778 A

- WO 10060653 A